# EUROPEAN PATENT SPECIFICATION

(11) **EP 1 790 373 B1**
(45) Date of publication and mention of the grant of the patent: **07.01.2009**
(21) Application number: 06023656.9
(22) Date of filing: 14.11.2006
(51) Int. Cl.: A61M 25/06, A61M 5/32

(54) **Safety indwelling needle**
Sicherheitsdauerverweilnadel
Aiguille de sécurité à demeure

(30) Priority: 28.11.2005 JP 2005341866
(43) Date of publication of application: 30.05.2007
(73) Proprietor: Covidien AG, 8212 Neuhausen am Rheinfall (CH)
(72) Inventor:
(74) Representative: Tomlinson, Edward James

(56) References cited:
- EP-A2- 1 291 035
- WO-A-91/01151
- US-A- 5 085 648
- US-A- 5 300 045

## Description

### Technical field

The present invention pertains to a type of indwelling needle that is used, for example in transfusion and blood collection for indwelling in the blood vessel after piercing it. The needle equipment includes means for protecting a user against accidental injury.

### Prior art

In the prior art, indwelling needles have been used in various applications, such as hemodialysis or feeding of prescribed medicine solutions, etc. The indwelling needle for use in this case has an external needle for the indwelling of the needle tip in the blood vessel of the patient and an internal needle that is inserted in the external needle to make smooth the operation of piercing the blood vessel of the patient. In the operation, while the internal needle is inserted in the external needle, and the tip portion of the internal needle protrudes a little from the tip portion of the external needle, together with the internal needle, the external needle pierces the blood vessel. Then, while the external needle indwells in the blood vessel, the internal needle is pulled out of the external needle (blood vessel), and the tube member or the like for feeding the prescribed blood or medicine solution or the like is connected to the rear end portion of the external needle, and hemodialysis or feeding of a medicine solution or the like is carried out.

A needle arrangement incorporating a needle tip cover is described in US 5,085,648. The needle tip cover is made of a tapered cylindrical body with an inner diameter larger on one end and smaller on the other end. The tip portion of the needle to be protected by said needle tip cover is formed in a size that permits it to be inserted into the opening on one end of the needle tip protective part but not into the opening on the other end. Consequently, when the internal needle is pulled with respect to the needle tip protective part while the rear side portion of the internal needle passes into the opening on the other end in the state in which the opening on one end faces the tip side of the internal needle, the needle tip protective part is coupled to the tip portion of the internal needle and the tip portion of the internal needle is covered.

However, for the aforementioned needle tip protective part of the prior art, although it cannot get from the tip side of the internal needle, it nevertheless is prone to shifting position on the rear side of the internal needle. Consequently, the tip portion of the internal needle may be exposed. Consequently, people have proposed a type of indwelling needle having a needle tip protective part that makes use of a spring member or the like to fix at the tip portion of the internal needle so that the tip portion of the internal needle can be reliably covered. However, for said indwelling needle, the number of parts increases, the manufacturing cost rises, and the resistance when the internal needle is pulled out of the external needle becomes higher, leading to poor operability. This is undesirable.

An example of an indwelling needle equipment incorporating a safety mechanism for an inner needle is described in US 2005/192536 A1. In the arrangement described therein, the inner needle is protected by a telescopic sheath attached to a needle hub.

### Summary of the invention

The purpose of the present invention is to solve the aforementioned problems of the prior art by providing a type of indwelling needle that has a simple structure, good operability, and can have the needle tip protective part mounted reliably on the tip portion of the internal needle.

In order to realize the aforementioned purpose, the present invention provides a type of indwelling needle equipment according to claim 1.

For the indwelling needle of the present invention with the aforementioned constitution, the needle tip protective part is composed of a cylindrical main body and a pair of movable gripped parts that are connected by a hinge to the edge portion of the window portions set facing each other on the peripheral surface of the main body. The other end portions, that is, the free ends, of the movable gripped parts are formed as the gripping portions that can grip each other. When the outer side portions of a pair of movable gripped parts are pressed to the inner side of the main body, the pair of gripping portions grip each other.

By means of gripping of the gripping portions of said pair of gripping parts, the interior of the main body becomes obstructed. In this case, the tip portion of the metal needle is covered with the needle tip protective part, and the safe state is maintained. Also, it is only required that the pair of movable gripped parts is connected in a rotatable way on the edge portions of the window portions. They may be formed integrated to the main body by means of an integrated molding. Consequently, the structure of the needle tip protective part becomes simpler, and it can be manufactured easily and with a high stability. Also, the edge portions for connecting with the pair of movable gripped parts may be either the edge portions of the front end side or the edge portions of the rear end side of the window portions.

As another characteristic feature of the indwelling needle of the present invention, the inner peripheral surface of the opening portion of said housing is formed such that the main body of said needle tip protective part can slide, and said pressing means is formed on the inner peripheral surface of said opening portion or the portion near it; when said needle tip protective part is positioned inside said housing, due to said metal needle, said two outer side portions are energized towards the outer side of said main body; when said needle tip protective part is taken out of said housing together with said internal needle, by the inner peripheral surface of said opening portion or the nearby portion, said two outer side portions are energized towards the inner side of said main body, and said pair of gripping portions is gripped on the tip end side of said metal needle.

For the indwelling needle of said type, when the internal needle is pulled out of the connection node in the state in which it is inserted in the external needle and the housing of the external needle, the internal needle has its tip portion gripped by the needle tip protective part so that it is pulled out of the needle tip protective part together with the external needle. In this case, by means of the inner peripheral surface of the opening portion of the housing or the portion near it, the outer side portions of the pair of movable gripped parts are energized to the inner side of the main body, and the pair of gripping portions grip each other. In this case, the gripping position of the pair of the gripping portions is near the tip portion of the metal needle in the internal needle, and when the internal needle is pulled out together with the needle tip protective part, the interior of the needle tip protective part is obstructed by the gripping portions of the pair of gripping parts.

Consequently, even when the needle tip protective part is moved to the rear side of the internal needle so that the tip portion of the internal needle is exposed, the tip portion of the metal needle is able to touch the gripping portions of the pair of gripping parts and the tip portion of the metal needle can't be exposed from the needle tip protective part. Consequently, there is no danger of harm caused by the tip portion of the metal needle, and the safety becomes higher. Also, in this case the operation for having the needle tip protective part gripped on the tip portion of the metal needle can be simply performed by pulling the internal needle with respect to the external needle. In this case, the resistance acting on the internal needle is only the reactive force acting from the inner peripheral surface of the opening portion or the portion near it when the pair of movable gripped parts is energized to the inner side of the main body. Consequently, it allows smooth operation.

As yet another characteristic feature of the constitution of the indwelling needle of the present invention, on the outer peripheral surface of the main body of said needle tip protective part, a ring member is set such that it can move in the axial direction, and said ring member is used to form said pressing means; as said ring member is moved from the rear side of said main body to the tip side, said ring member has said two outer side portions energized to the inner side of said main body, so that said pair of gripping portions is gripped, and the interior of said main body is obstructed. In this case, movement of the ring member can be performed by the operator. As another scheme, when the internal needle is pulled from the external needle, at a prescribed portion of the external needle, movement of the ring member together with the internal needle is stopped.

As yet another characteristic feature of the constitution of the indwelling needle of the present invention, one end portion of said pair of movable gripped parts is connected to one edge portion of said window portions at positions which deviate from each other in the axial direction. As a result, one end portions of a pair of movable gripped parts are controlled in the rear side portions of the window portions at portions diverge from each other in the axial direction, so that the force is dispersed and the resistance becomes smaller when the pair of movable gripped parts is energized to the inner side of the main body. As a result, since the resistance is small, the operation can be performed smoothly. In addition, by delaying the timing for energizing the movable gripped part on one side and the movable gripped part on the other side towards the inner side of the main body, it is possible to realize diversity in the shape of the gripping parts, and it is possible to form the gripping parts in a simple structure. Also, because the timing for energizing the pair of movable gripped parts towards the interior of the main body is delayed, the movable gripped part on one side and the movable gripped part on the other side are moved stepwise in the interior of the main body. As a result, the stroke becomes stable when the pair of movable gripped parts is gripped.

As yet another characteristic feature in the constitution of the indwelling needle of the present invention, one of the gripping portions of said pair of movable gripped parts is formed as a protrusion, and the other gripping portion is formed as a recess which grips by said protrusion. In this case, the recess may be the recess having its periphery surrounded by the inner wall surface, or the recess formed between two pieces sandwiched between the two sides of the protrusions. As a result, it is possible to grip reliably with a simple structure.

As yet another characteristic feature in the constitution of the indwelling needle of the present invention, this invention provides a type of indwelling needle characterized by the following facts: the indwelling needle is composed of an external needle consisting of a tubular cannula and a housing that is connected to the base end portion of said cannula and has a space connected to the interior of said cannula, an internal needle consisting of a tubular metal needle that has a tip portion protruding from the tip opening portion of said cannula and can be inserted in said external needle, and a hub connected to the base end portion of said metal needle, and a needle tip protective part, which has an insertion hole into which the rear side portion of said metal needle can be inserted and is mounted on said housing while the rear side portion of said metal needle is inserted in said insertion hole, and which is taken out of said external needle together with said internal needle while the tip portion of said metal needle is engaged in said insertion hole when said internal needle is pulled out of said external needle; the main body of said needle tip protective part is formed in a cylindrical shape; on the peripheral surface of said main body, window portions extending in the axial direction of said main body are formed on the facing portions, respectively; and at the same time, in one of the edge portions in the longitudinal direction of said window portions, a pair of movable gripped parts is set; one end portion of said movable gripped parts is connected by a hinge on said edge portion; when said metal needle is inserted in said main body, the outer side portions protrude out of said main body; and the other end portions of said movable gripped parts are formed on flexible gripping pieces; when said metal needle is out of the portion of said main body corresponding to said pair of movable gripped parts, the two outer side portions of said pair of movable gripped parts are pressed to the internal side of said main body by a pressing means, so that said two gripping pieces grip the other edge portions of said window portions, and the interior of said main body is obstructed.

For the indwelling needle with said constitution, when the gripping pieces of the pair of movable gripped parts are gripped on the edge portions of the window portions, the interior of the main body is obstructed. Consequently, there is no need to have the gripping pieces grip each other, and the force for energizing the pair of movable gripped parts towards the inner side of the main body can be smaller. That is, it is only required that the state in which a pair of the gripping pieces is positioned in the inner side of the main body is maintained as the gripping pieces are bent as they enter the inner side of the main body, and the gripping pieces grip the edge portions of the window portions when a pair of movable gripped parts is energized towards the inner side of the main body. In this case, when there is a force for energizing the gripping pieces towards the outer side of the main body, it is only required that the gripping pieces grip the edge portions of the window portions, and the gripping pieces may be at a position away from the edge portions of the window portions. That is, for the movable gripped parts, by simply connecting them to the edge portions of the window portions with a hinge, they can be formed in a state in which there is no energizing in any direction. Consequently, as long as no specific energizing force is applied, the state in which the gripping pieces are positioned on the inner side of the edge portions of the window portions is maintained.

In this case, the inner peripheral surface of the opening portion of the housing is formed such that the main body of the needle tip protective part can slide, and the pressing means is formed from the inner peripheral surface of the opening portion or the portion near it. When the needle tip protective part is positioned in the housing, the two outer side portions are energized towards the outer side of the main body by means of the metal needle. When the needle tip protective part is taken out of the housing together with the internal needle, by means of the inner peripheral surface of the opening portion or the portion near it, the two outer side portions are energized towards the inner side of the main body, and the pair of the gripping pieces can grip the edge portions on the other side of the window portions on the tip side of the opening portion. Also, on the outer peripheral surface of the main body of the needle tip protective part, the ring member is set such that it can move in the axial direction, and the ring member forms the pressing means. As the ring member is moved from the rear side of the main body to the tip side, the ring member energizes the two outer side portions towards the inner side of the main body, the pair of gripping pieces grip the edge portions on the other side, and the interior of the main body is obstructed.

As yet another characteristic feature of the constitution of the indwelling needle of the present invention, said window portions are formed in nearly the central portion in the axial direction of said needle tip protective part. As a result, when the needle tip protective part is pulled from the housing, due to contact between the outer side portions of the movable gripped parts and the inner peripheral surface of the housing, the needle tip protective part is not going to move in an unstable condition due to the loss of balance. As a result, it is possible to pull the needle tip protective part together with the internal needle from the external needle in a smooth operation. For example, if the window portions are formed on the front end side of the axial direction of the needle tip protective part, when almost all of the needle tip protective part is pulled out of the housing, the window portions would interfere with the inner peripheral surface of the opening portions of the housing, so that the needle tip protective part cannot be fully pulled out in a smooth operation. Now, according to the present invention, since the window portions are formed nearly on the central portion of the axial direction of the needle tip protective part, it is possible to pull out the needle tip protective part together with the internal needle from the external needle in a smooth operation.

### Brief description of the figures

The present invention will now be described, by way of example only, with reference to the accompanying drawings in which:
Figure 1 shows the indwelling needle of Embodiment 1. (a) is a side view, and (b) is a front view;
Figure 2 is a cross-sectional view of the indwelling needle;
Figure 3 is a cross-sectional view illustrating the state in which the internal needle is being pulled out of the external needle, and the movable held portion is energized to the main body side;
Figure 4 is a cross-sectional view illustrating the state in which the internal needle is being pulled out of the external needle, and the movable holding portion is energized to the main body side;
Figure 5 is a cross-sectional view illustrating the state in which the internal needle has been pulled from the external needle;
Figure 6 illustrates the needle tip protective part in the indwelling needle in Embodiment 1. Figure 6(a) is a plane view; (b) is a side view; (c) is a front view; (d) is an inner side view; and (e) is a bottom view;
Figure 7 is a cross-sectional view taken across 7-7 in Figure 6(a);
Figure 8 illustrates the state in which the held portion and holding portion of the needle tip protective part shown in Figure 6(a) is a plane view; (b) is a side view; (c) is a front view; (d) is an inner side view; and (e) is a bottom view;
Figure 9 is a cross-sectional view taken across 9-9 of Figure 8(a);
Figure 10 is a cross-sectional view of the indwelling needle in Embodiment 2 of the present invention;
Figure 11 is a cross-sectional view illustrating the state in which the internal needle is being pulled from the external needle of the indwelling needle shown in Figure 10, and the held portion and the holding portion grip each other;
Figure 12 is a cross-sectional view illustrating the state in which the internal needle has been pulled from the external needle of the indwelling needle shown in Figure 10;
Figure 13 illustrates the needle tip protective part of the indwelling needle in Embodiment 2. (a) is a plane view; (b) is a side view; (c) is a front view; (d) is an inner side view; (e) is a bottom view;
Figure 14 is a cross-sectional view taken across 14-14 in Figure 13(a);
Figure 15 illustrates the state in which the held portion and holding portion of the needle tip protective part shown in Figure 13 grip each other. (a) is a plane view; (b) is a side view; (c) is a front view; (d) is an inner side view; (e) is a bottom view;
Figure 16 is a cross-sectional view taken across 16-16 of Figure 15(a);
Figure 17 is a cross-sectional view of the indwelling needle in Embodiment 3 of the present invention;
Figure 18 is a cross-sectional view illustrating the state after the internal needle is being pulled out of the external needle in the indwelling needle shown in Figure 17;
Figure 19 is a cross-sectional view illustrating the state after the internal needle has been pulled out of the external needle in the indwelling needle shown in Figure 17;
Figure 20 illustrates the needle tip protective part of the indwelling needle in Embodiment 3. (a) is a plane view; (b) is a side view; (c) is a front view; (d) is an inner side view; (e) is a bottom view;
Figure 21 is a cross-sectional view taken across 21-21 in Figure 20(a);
Figure 22 illustrates the state in which the gripping pieces of the needle tip protective part shown in Figure 20 have entered the main body. (a) is a plane view; (b) is a side view; (c) is a front view; (d) is an inner side view; (e) is a bottom view;
Figure 23 is a cross-sectional view taken across 23-23 in Figure 22(a);
Figure 24 is a diagram illustrating the needle tip protective part of the indwelling needle in Embodiment 4. (a) is a plane view; (b) is a side view; (c) is a front view; (d) is an inner side view; (e) is a bottom view;
Figure 25 is a cross-sectional view taken across 25-25 of Figure 24(a);
Figure 26 is a side view illustrating the state in which the ring-shaped member is positioned on the rear side of the main body;
Figure 27 is a side view illustrating the state in which the ring-shaped member is moved to the front side of the main body, and one movable gripped part is energized in the main body;
Figure 28 is a side view illustrating the state in which the ring-shaped member is moved to the front side of the main body, and the pair of movable gripped parts are energized in the main body;
Figure 29 is a side view illustrating the state in which the ring-shaped part is further moved, and the pair of movable gripped parts are energized in the main body;
Figure 30 is a side view illustrating the state in which the ring-shaped part is made to pass through the portion corresponding to the pair of movable gripped parts in the main body, and the pair of movable gripped parts is gripped; and
Figure 31 is a side view illustrating the state in which the ring-shaped member is moved to the front side of the main body, and the gripping state of the pair of the movable gripped parts is maintained.

### Preferred Embodiments of the invention

### Embodiment 1

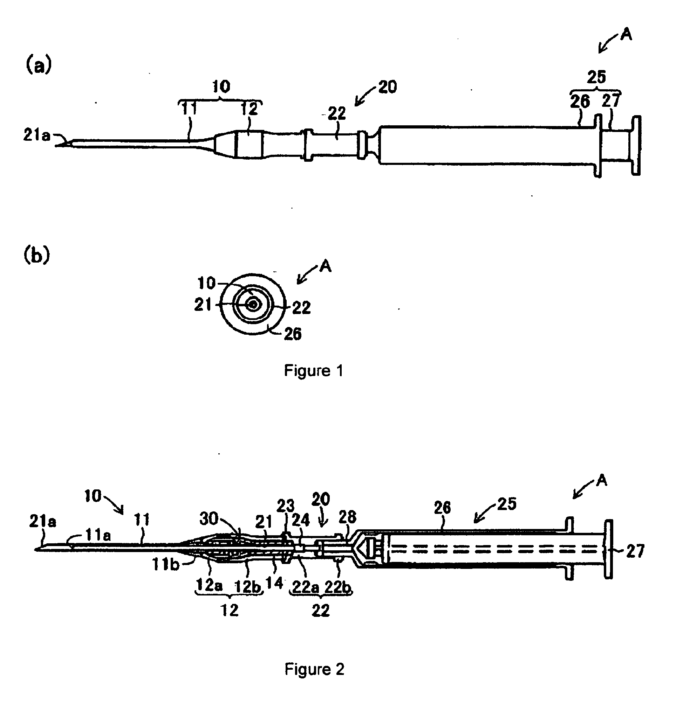

In the following, a detailed explanation will be given regarding the indwelling needle of the present invention with reference to figures. Figures 1-5 illustrate indwelling needle A in Embodiment 1 of the present invention. This indwelling needle A is composed of external needle (10) that indwells in the blood vessel of the patient, internal needle (20) that can be inserted into the interior of external needle (10) and has a sharp tip portion which can pierce the body of the patient, suction part (25) mounted on the rear portion of internal needle (20), and needle tip protective part (30) for protecting the tip portion of internal needle (20). Said external needle (10) is composed of a fine tubular cannula (11), and housing (12) connected to the base end portion of cannula (11) (the right hand side in Figure 1 refers to the rear end portion. In the following, the tip side (left hand side) of cannula (11) will be referred to as the front side, and the base end side (right hand side) of cannula (11) will be referred to as the rear side in the explanation).

Said cannula (11) has internal cavity (13) that forms the flow channel that goes from the tip portion through to the rear end portion. On the outer peripheral surface of tip portion (11 a) of cannula (11), a taper is formed with the front end side tapered finer. Also, rear end portion (11b) of cannula (11) is formed with the rear end side gradually widens. This cannula (11) indwells in the blood vessel of the patient for withdrawing blood from the blood vessel, or for feeding medicine solutions, etc., into the blood vessel. Also, housing (12) is composed of housing front portion (12a) fixed on rear end portion (11b) of cannula (11), and housing rear portion (12b) that forms the rear side portion of housing (12), and it is formed in a nearly cylindrical shape.

The front end portion of housing front portion (12a) is formed in a slender shape with a smooth surface formed between the housing front portion (12a) and rear end portion (11b) of cannula (11). Also, the front side portion of housing rear portion (12b) is formed in the same diameter as that of the rear end portion of housing front portion (12a), opening portion (14) that forms the rear side of housing rear portion (12b) is formed finer than the front side portion of housing rear portion (12b), and wider than the front end portion of housing front portion (12a). In addition, opening portion (14) is formed in female Luer syringe shape with the diameter gradually increasing from the front to the rear. Then, in the portion between the front side portion of housing rear portion (12b) and opening portion (14), it is formed in a cylindrical shape with the diameter decreasing toward the rear side.

Consequently, in the interior of housing (12), space (15a) with a larger inner diameter is formed for the portion from the front side portion to nearly the central portion along the axial direction, and space (15b) with a little smaller inner diameter is formed as the rear side portion. The portion of the inner peripheral surface of housing (12) between space (15a) and space (15b) is formed as tapered slope surface (16). Also, the front side portion of space (15a) also gradually enlarges from the front portion to the rear portion along the shape of housing front portion (12a).

Said internal needle (20) is composed of metal needle (21) made of stainless steel and hub (22) fixed on the base end portion (the rear end portion positioned on the right hand side as shown in the figure) of metal needle (21). Said metal needle (21) is made of a fine tubular injection needle, with front end portion (21 a) cut oblique with respect to the axial direction to form a sharp portion. Also, the front side portion of metal needle (21) is formed a little larger than the rear side portion of metal needle (21), with step (21 b) formed at the boundary portion. This metal needle (21) works to ensure smooth insertion when tip portion (11 a) of cannula (11) is inserted into a patient's blood vessel. It is inserted into external needle (10) from the rear end portion of external needle (10), and front end portion (21 a) protrudes out of the opening of tip portion (11 a) of cannula (11) for use. In this case, front end portion (21 a) of metal needle (21) passes through the interior of housing (12) and the interior of cannula (11), and it protrudes out of tip portion (11a) of cannula (11).

Said hub (22) is the portion that works as a holding portion for holding internal needle (20). It is fixed on metal needle (21) such that the outer peripheral portion of the rear end portion of metal needle (21) is covered. This hub (22) is composed of hub main body (22a) that fixes metal needle (21), and cylindrical mounting portion (22b) which is formed in the rear side of hub main body (22a) and has a recess opening towards the rear side. Also, mounting portion (22b) is formed in a female Luer syringe shape, and it gradually becomes larger from the front side to the rear side.

Flange-shaped collar portion (23) is formed on the front end peripheral portion of hub main body (22a). Also, metal needle (21) is bonded and fixed via bonding portion (24) to hub main body (22a). Suction part (25) is made of a syringe. It has piston part (27) inserted in a movable state in cylinder part (26) that can accommodate gas or liquid. Then, as male Luer syringe (28) that forms the tip portion of cylinder part (26) is inserted into mounting portion (22b) of hub (22), suction part (25) is assembled on internal needle (20).

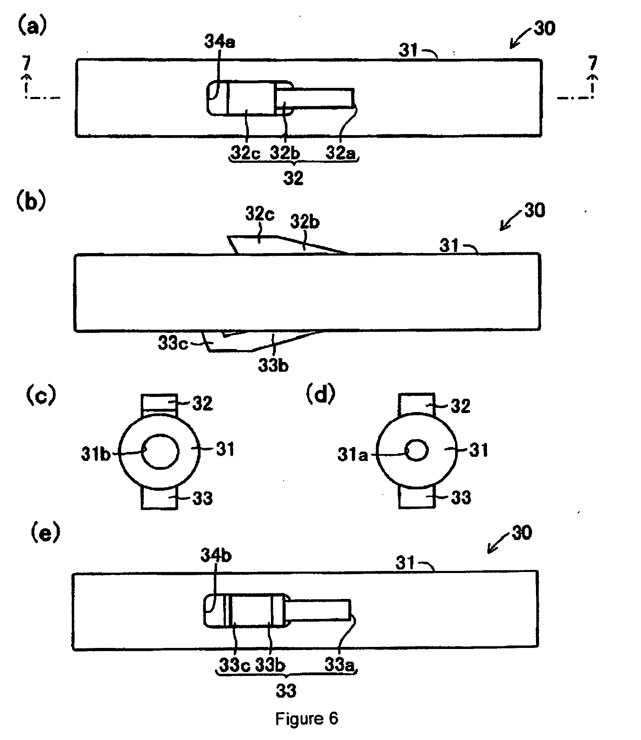

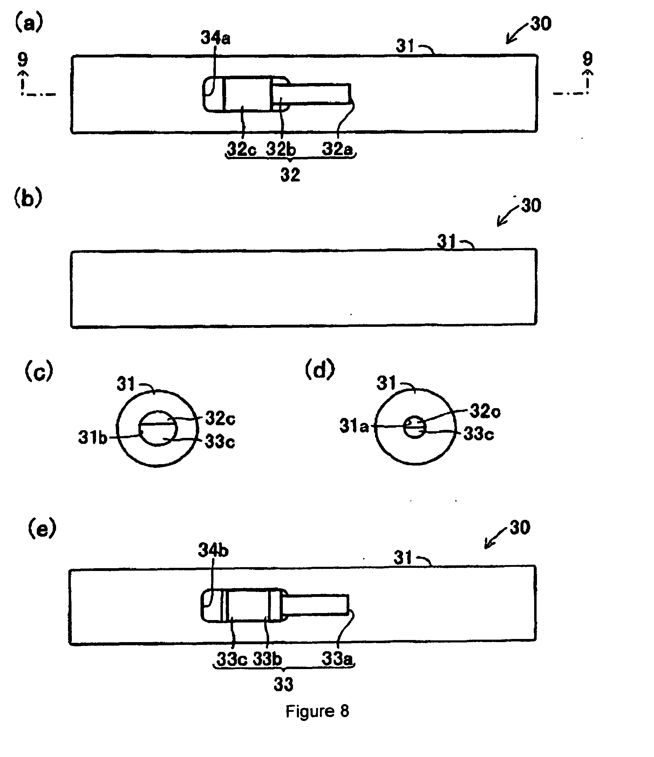

Also, needle tip protective part (30) is set in housing (12) of external needle (10). As shown in Figures 6-9, said needle tip protective part (30) is composed of main body (31) and a pair of movable gripped parts of the present invention, that is, movable held part (32) and movable holding part (33). Said main body (31) is formed in a cylindrical shape. The inner peripheral portion of its rear end portion (or the end portion positioned on the side of opening portion (14) when set in housing (12)) has a fine-diameter portion (31 a) with a diameter finer than that of the other portion formed there. A step is formed between fine-diameter portion (31 a) and front side peripheral surface (31 b). This fine-diameter portion (31 a) is formed with a size that stops the tip side portion of metal needle (21) yet allows passage of the remaining portion of metal needle (21) other than the tip side portion.

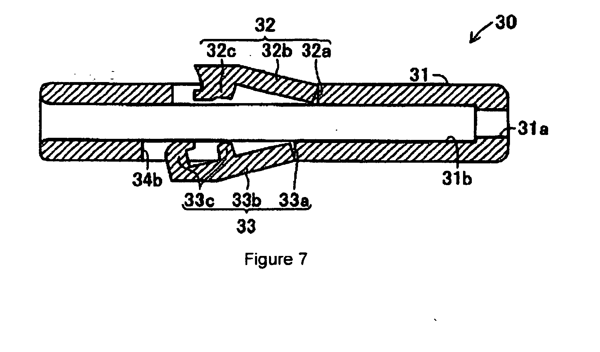

Window portions (34a), (34b) extending in the axial direction and at positions which deviate a little from each other in the axial direction are formed at portions facing each other and a little shifted to the front side portion with respect to the center in the axial direction of main body (31). Said window portions (34a), (34b) are each formed as holes with a larger width of the front side and a smaller width of the rear side. Base end portion (32a) of movable held part (32) is connected by a hinge, preferably a living hinge, to the rear end edge portion of window portion (34a), and base end portion (33a) of movable holding part (33) is connected by a hinge, preferably a living hinge, to the rear end edge portion of window portion (34b). In the state shown in Figures 6 and 7, when needle tip protective part (30) is set in housing (12), outer side portion (32b) of movable held part (32) protrudes towards the outer side along slope surface (16). Also, held portion (32c) of the tip is formed wider than base end portion (32a), and the inner surface of held portion (32c) is formed on the protrusion that protrudes towards the inner side of main body (31).

In the state shown in Figures 6 and 7, when needle tip protective part (30) is set in housing (12), outer side portion (33b) of movable holding part (33) protrudes along slope surface (16) of housing (12) towards the outer side, and holding portion (33c) of the tip is formed wider than base end portion (33a). Then a pair of protrusions is formed on the inner surface of holding portion (33c). They protrude towards the inner side of main body (31), and can be fixed by holding held portion (32c) of movable held part (32) from the front and back. In the state shown in Figures 6 and 7, metal needle (21) can be inserted through the portion inside needle tip protective part (30) corresponding to movable held part (32) and movable holding part (33).

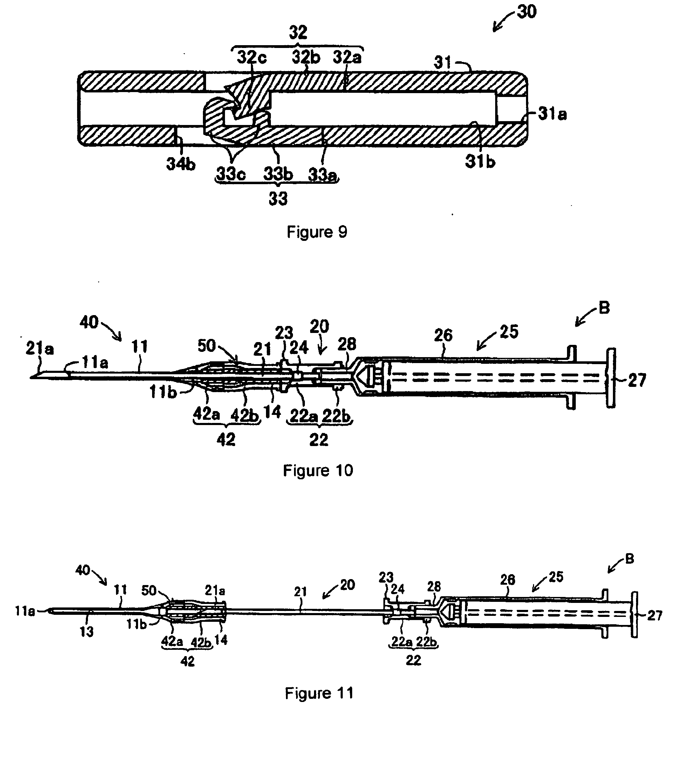

Then, when metal needle (21) is positioned inside needle tip protective part (30), held portion (32c) and holding portion (33c) are in contact with the outer peripheral surface of metal needle (21), and outer side portion (32b) of movable held part (32) and outer side portion (33b) of movable holding part (33) protrude towards the outer side of main body (31). Also, metal needle (21) is not located at the portion inside needle tip protective part (30) corresponding to movable held part (32) and movable holding part (33), and, when outer side portion (32b) and outer side portion (33b) are pressed to the inner side of main body (31) and are at the same position as the peripheral surface of main body (31), as shown in Figure 9, held portion (32c) and holding portion (33c) grip each other.

Then, while metal needle (21) of internal needle (20) passes through needle tip protective part (30) set in housing (12) of external needle (10), and the rear end side of metal needle (21) is positioned inside needle tip protective part (30), front end portion (21 a) of metal needle (21) is made to protrude a little from tip portion (11a) of cannula (11), so that indwelling needle A shown in Figures 1 and 2 is obtained. In this case, collar portion (23) of internal needle (20) comes into contact with the rear end of opening portion (14) in housing (12) of external needle (10).

In this constitution, when indwelling needle A is applied, first of all, front end portion (21 a) of metal needle (21), tip portion (11a) of cannula (11) in indwelling needle A in the state shown in Figures 1 and 2 are simultaneously pushed to pierce a portion of the patient, such as the wrist, to reach a blood vessel. Then, piston part (27) of suction part (25) attached on the rear portion of hub (22) is pulled back from cylinder part (26). As a result, the blood in the blood vessel seeps into metal needle (21), and it enters the portion in metal needle (21) corresponding to hub main body (22a) of hub (22). Then, the blood is sucked into cylinder part (26). As a result, the color becomes red inside hub (22) and cylinder part (26), and the operator can find out that front end portion (21 a) of metal needle (21) has reached the blood vessel.

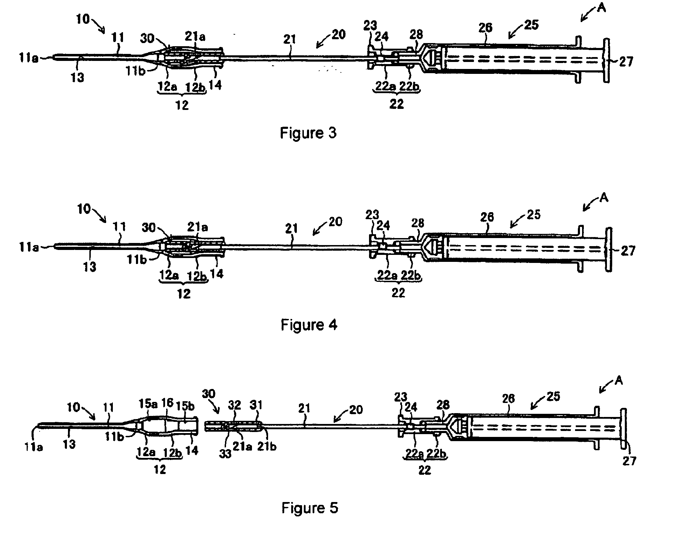

Then, while internal needle (20) is kept as it is, external needle (10) is pressed forward a little, so that tip portion (11 a) of external needle (10) further enters the front portion within the blood vessel. Then, while tip portion (11a) of external needle (10) is kept in the blood vessel of the patient, as shown in Figure 3, together with suction part (25), internal needle (20) is being pulled toward the rear side of external needle (10). Because of this metal needle (21) falls back from cannula (11) and front end portion (21 a) of metal needle (21) is positioned inside needle tip protective part (30) within housing (12). Then, together with suction part (25), internal needle (20) is pulled back to the rear side of external needle (10), and, as step (21 b) of metal needle (21) reaches fine-diameter portion (31 a) of needle tip protective part (30), metal needle (21) and needle tip protective part (30) grip each other via step (21 b) and fine-diameter portion (31 a).

Then, together with suction part (25), internal needle (20) is pulled back to the rear side of external needle (10). As a result, while needle tip protective part (30) grips the front end portion of metal needle (21), internal needle (20) retreats together with suction part (25) from external needle (10). In this case, when metal needle (21) is still positioned in the portion inside needle tip protective part (30) corresponding to movable held part (32) and movable holding part (33), by means of metal needle (21), movable held part (32) and movable holding part (33) are energized to the outer side of main body (31), and they are set along slope surface (16) within housing (12). Then, as needle tip protective part (30) grips metal needle (21), they start moving towards the outer side of housing (12). As a result, first of all, by means of the rear side portion of slope surface (16), outer side portion (32b) of movable held part (32) is energized to the inner side of main body (31), and the state becomes that shown in Figure 3.

Then, by means of the rear side portion of slope surface (16), outer side portion (33b) of movable holding part (33) is energized to the inner side of main body (31), and the state becomes that shown in Figure 4. Then, when movable held part (32) and movable holding part (33) pass on slope surface (16) and enter space (15b) of housing (12), held portion (32c) and holding portion (33c) grip each other entirely. In this case, held portion (32c) of movable held part (32) and/or holding portion (33c) of movable holding part (33) are flexible, and, as the flexible portions bend, held portion (32c) and holding portion (33c) can grip each other.

Then, as internal needle (20) is pulled back together with suction part (25) to the rear side of external needle (10), while needle tip protective part (30) grips the tip of metal needle (21), internal needle (20) retreats together with suction part (25) from external needle (10), and the state becomes that shown in Figure 5. In this case, front end portion (21 a) of metal needle (21) becomes positioned between fine-diameter portion (31 a) and the portion in needle tip protective part (30) where held portion (32c) and holding portion (33c) grip each other. Consequently, there is no way that the operator can make contact with front end portion (21 a) of metal needle (21) and get hurt. That is, safety is guaranteed.

Then, the connecting portion of a tube member (not shown in the figure) extending from the feeding device (not shown in the figure) of the medicine solution or the like is connected to opening portion (14) of housing (12). In this way, while the tube member is connected to housing (12), the feeding device connected to the base end portion of the tuning means operates, and the medicine solution, etc., is fed to the blood vessel. As explained above, indwelling needle A can be used. On the other hand, internal needle (20) pulled out of external needle (10) is discarded at a prescribed site.

In this way, in indwelling needle A of the present embodiment, needle tip protective part (30) is composed of main body (31) in a cylindrical shape as well as movable held part (32) and movable holding part (33) that are connected by a hinge to the edge portions of window portions (34a), (34b) set facing each other on the peripheral surface of main body (31). Then, when metal needle (21) is put in needle tip protective part (30), by means of metal needle (21), movable held part (32) and movable holding part (33) are energized towards the outer side of main body (31). Also, after metal needle (21) has been pulled back from the portion of needle tip protective part (30) corresponding to movable held part (32) and movable holding part (33), as needle tip protective part (30) is pulled out together with internal needle (20) from external needle (10), by means of slope surface (16) of housing (12), movable held part (32) and movable holding part (33) are energized towards the inner side of main body (31).

The structure is such that when needle tip protective part (30) is pulled out of housing (12), held portion (32c) of movable held part (32) and holding portion (33c) of movable holding part (33) grip each other, and the front end portion of main body (31) is obstructed. Consequently, even when needle tip protective part (30) is moved to the rear side of internal needle (20) so that front end portion (21 a) of metal needle (21) that has been removed from external needle (10) is exposed, by simply bringing front end portion (21a) of metal needle (21) into contact with the gripping portion between held portion (32c) and holding portion (33c), front end portion (21 a) of metal needle (21) cannot be exposed. Consequently, there is no way that the operator can make contact with front end portion (21 a) of metal needle (21) and get hurt. That is, safety is guaranteed. Also, the operation in this case is simple because it is only required that internal needle (20) be pulled with respect to external needle (10).

Also, in this case, the resistance to internal needle (20) is only the reactive force acting from slope surface (16) of housing (12) when movable held part (32) and movable holding part (33) are energized to the inner side of main body (31). Consequently, smooth operation can be performed. Also in this case, it is only required that movable held part (32) and movable holding part (33) be connected in a rotatable way to the edge portions of window portions (34a), (34b), so that the connection can be performed even without elastic and other characteristics. Also, movable held part (32) and movable holding part (33) can be formed integrated to main body (31) by means of integrated molding. Consequently, the structure of needle tip protective part (30) becomes simpler, and manufacturing can be performed easily and with a high stability.

### Embodiment 2

Figures 10-12 illustrate indwelling needle B in Embodiment 2 of the present invention. For this indwelling needle B, housing (42) of external needle (40) is composed of housing front portion (42a) that forms the front side portion of housing (42) and housing rear portion (42b) that forms the rear side portion of housing (42), and it is formed in a nearly cylindrical shape. Then, in the interior of housing (42), space (45a) with a larger inner diameter is formed in the portion from the front side portion of housing front portion (42a) to nearly the center of housing rear portion (42b) is formed, and the rear side portion is formed in space (45b) with an inner diameter a little smaller than that of the front side portion of space (45a). Also, space (45b) is formed such that its inner diameter gradually increases from the front side to the rear side. Then, tapered slope surface (46), short in the axial direction, is formed between space (45a) and space (45b) on the inner peripheral surface of housing (42).

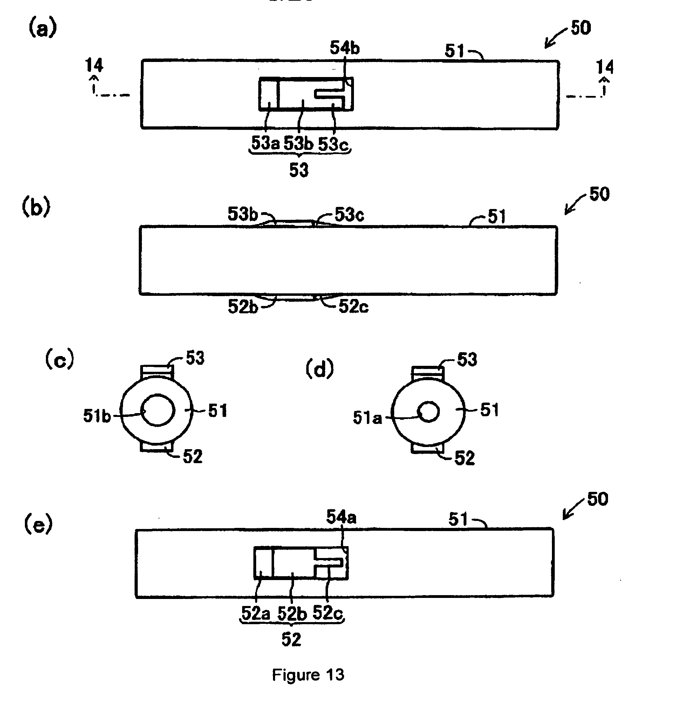

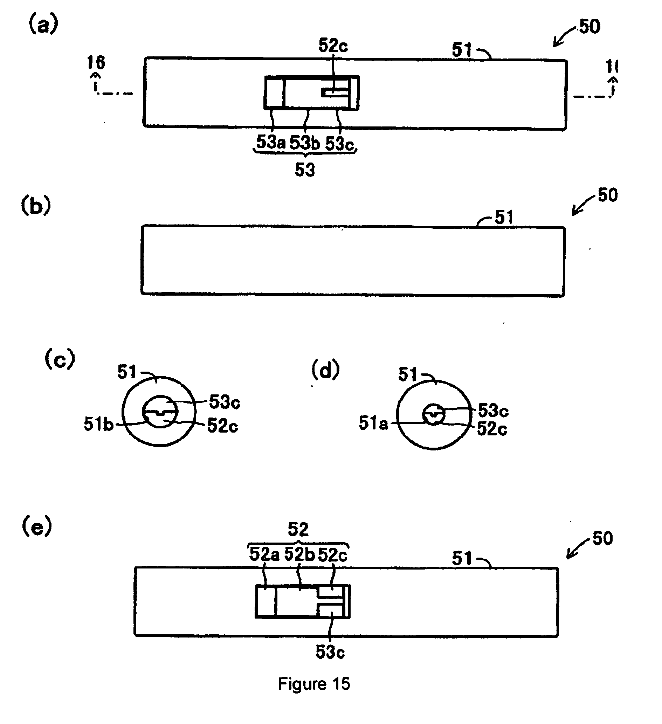

As shown in Figures 13-16, needle tip protective part (50) of indwelling needle B is composed of main body (51), movable held part (52) and movable holding part (53). Said main body (51) is formed in a cylindrical shape, and the inner peripheral surface of its rear end portion (the end portion positioned on the rear end opening side when it is set in housing (42)) is formed with a diameter smaller than the remaining portion to form fine-diameter portion (51a), and a step is formed between fine-diameter portion (51 a) and the remaining front-side peripheral surface (51 b). Also, window portions (54a), (54b) extending in the axial direction are formed in the portions facing each other at the site a little ahead of the center in the axial direction of main body (51). Both said window portions (54a), (54b) are formed as rectangular holes.

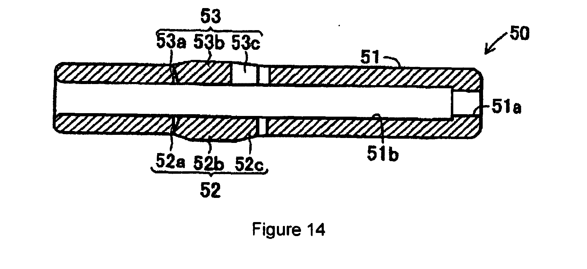

Then, base end portion (52a) of movable held part (52) is connected by a hinge to the front end edge portion of window portion (54a), and base end portion (53a) of movable holding part (53) is connected by a hinge to the front end edge portion of window portion (54b). In the state shown in Figures 13 and 14, outer side portion (52b) of movable held part (52) protrudes along slope surface (46) of housing (42) towards the outer side. Also, held portion (52c) of the rear end (free end) is formed as a protrusion with a width narrower than that of movable held part (52).

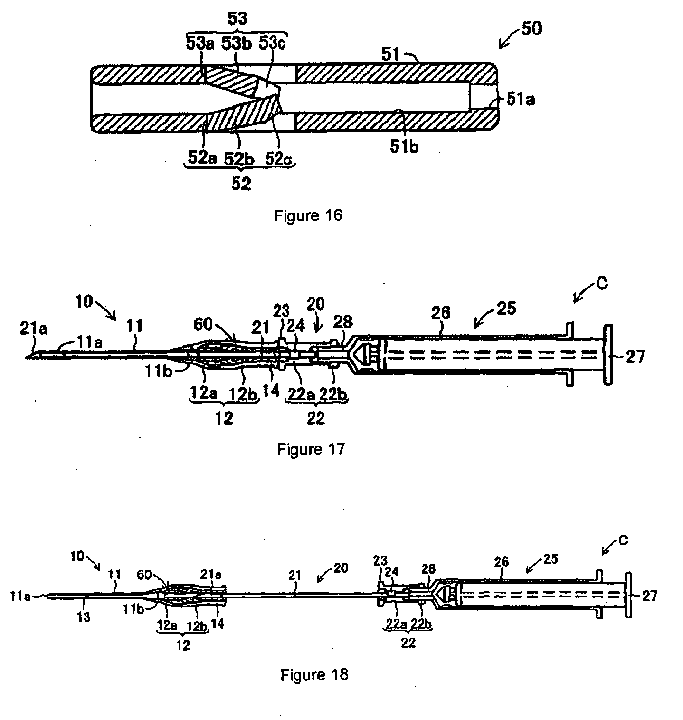

In the state shown in Figures 13 and 14, outer side portion (53b) of movable holding part (53) protrudes towards the outer side along slope surface (46) of housing (42). Holding portion (53c) on the rear end is formed as a pair of protrusions formed by providing a recess that has the rear side and upper/lower sides open at the center in the width direction. This holding portion (53c) is formed such that held portion (52c) of movable held part (52) can be fixed by holding from the left/right sides (front/rear sides in the state shown in Figures 14 and 16). The constitution of the remaining portion of said indwelling needle B is the same as that in said indwelling needle A. Consequently, the same part numbers as those adopted for indwelling needle A are adopted, and they will not be explained again.

When said indwelling needle B is in use, the same operation as that for indwelling needle A is performed. In this case also after front end portion (21 a) of metal needle (21), tip portion (11 a) of cannula (11) in indwelling needle B in the condition shown in Figure 10 are simultaneously is pushed to pierce the wrist of the patient to reach the blood vessel external needle (10) is pushed a little more to the front side so that tip portion (11 a) of external needle (10) reaches the blood vessel. Then, while tip portion (11a) of external needle (10) indwells in the blood vessel of the patient as shown in Figure 11, (25), internal needle (20) is pulled towards the rear side of external needle (10) together with suction part. Then, metal needle (21) retreats from cannula (11) and front end portion (21 a) of metal needle (21) is positioned in needle tip protective part (50) in housing (42). Then, internal needle (20) is pulled towards the rear side, and step (21 b) of metal needle (21) and fine-diameter portion (51 a) of needle tip protective part (50) grip each other.

Then, together with suction part (25), internal needle (20) is pulled back to the rear side of external needle (10). As a result, while needle tip protective part (50) grips the front end portion of metal needle (21), internal needle (20) retreats together with suction part (25) from external needle (10). In this case, when metal needle (21) is still positioned in the portion inside needle tip protective part (50) corresponding to movable held part (52) and movable holding part (53), by means of metal needle (21), movable held part (52) and movable holding part (53) are energized to the outer side of main body (51), and they are set along slope surface (16) within housing (42). Then, as needle tip protective part (50) grips metal needle (21), they start moving towards the outer side of housing (42). As a result, first of all, outer side portion (52b) of movable held part (52) and outer side portion (53b) of movable holding part (53) are energized by slope surface (46) towards the inner side of main body (51), and the state becomes that shown in Figure 11.

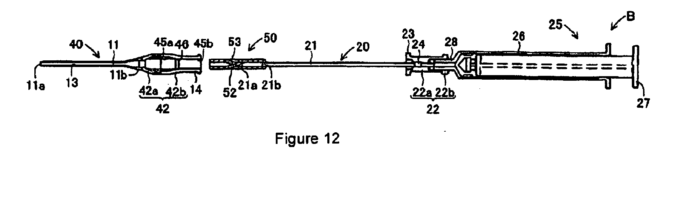

Then, when movable held part (52) and movable holding part (53) pass on slope surface (46) and enter space (45b) of housing (42), held portion (52c) and holding portion (53c) grip each other entirely. Then, as internal needle (20) is pulled back together with suction part (25) to the rear side of external needle (10), while needle tip protective part (50) grips the tip of metal needle (21), internal needle (20) retreats together with suction part (25) from external needle (10), and the state becomes that shown in Figure 12. Then, the connecting portion of a tube member extending from the feeding device of the medicine solution or the like is connected to opening portion (14) of housing (42). In this way, the feeding device connected to the base end portion of the tuning means operates, and the medicine solution, etc., is fed to the blood vessel.

For indwelling needle B, held portion (52c) is formed on the rear end portion of movable held part (52), and holding portion (53c) is formed on the rear end portion of movable holding part (53). Consequently, the distance between the portion where held portion (52c) and holding portion (53c) are gripped and the front end portion of main body (51) becomes longer, and front end portion (21 a) of metal needle (21) moves away from the front end portion of main body (51). As a result, front end portion (21 a) of metal needle (21) can barely exit the front end portion of needle tip protective part (50). Also, when needle tip protective part (50) is moved to the rear side of internal needle (20) so as to expose front end portion (21 a) of metal needle (21), front end portion (21a) of metal needle (21) presses the gripped portion between held portion (52c) and holding portion (53c), so that the grip between held portion (52c) and holding portion (53c) is made stronger. Consequently, safety is further improved. The other functions and effects of indwelling needle B are the same as those of said indwelling needle A.

### Embodiment 3

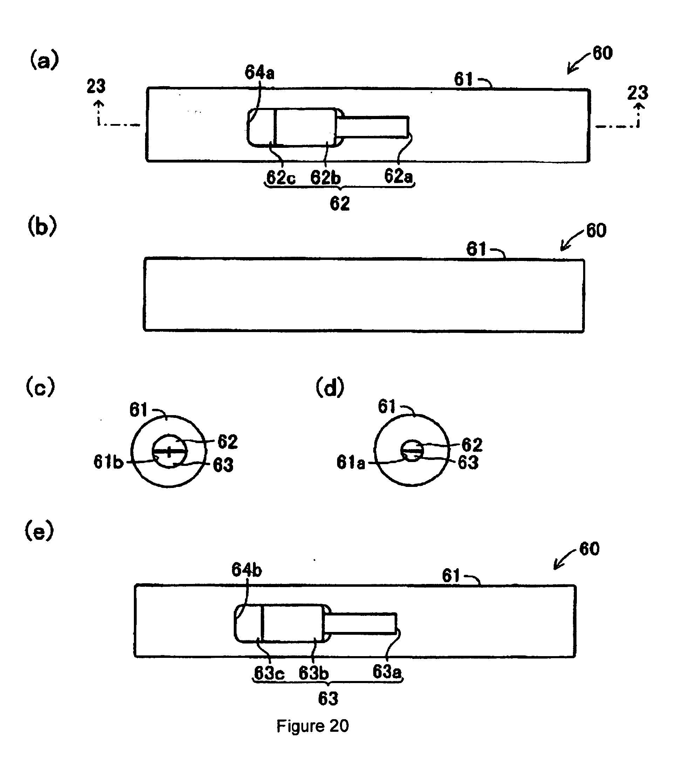

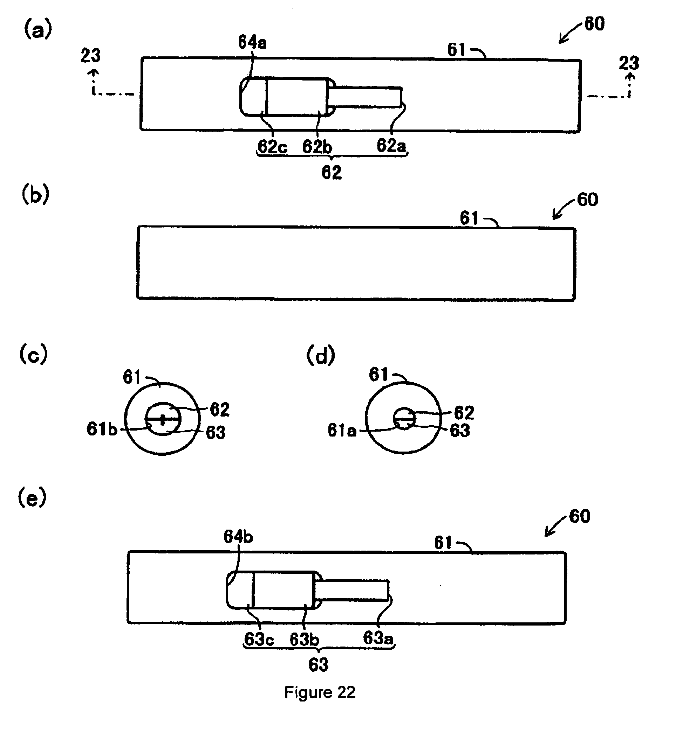

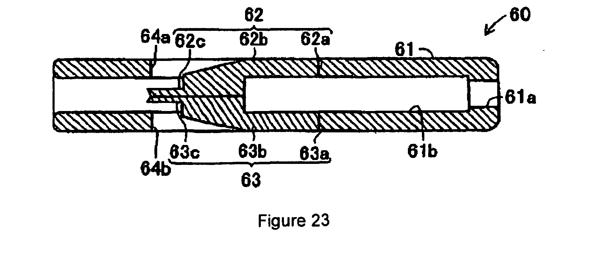

Figures 17-19 illustrate indwelling needle C in Embodiment 3 of the present invention. For this indwelling needle C, as shown in Figures 20-23, needle tip protective part (60) is composed of main body (61) and a pair of movable gripped parts (62), (63). Said main body (61) is formed in a cylindrical shape, and the inner peripheral surface of its rear end portion (the right hand side end portion shown in Figures 20-23) is formed with a diameter smaller than the remaining portion to form fine-diameter portion (61 a), and a step is formed between fine-diameter portion (61 a) and the remaining front-side peripheral surface (61 b). Also, window portions (64a), (64b) extending in the axial direction are formed in the portions facing each other at the site a little ahead of the center in the axial direction of main body (61). Both said window portions (54a), (54b) [sic; 64a, 64b] are formed as tapered holes wider in front and narrower toward the rear.

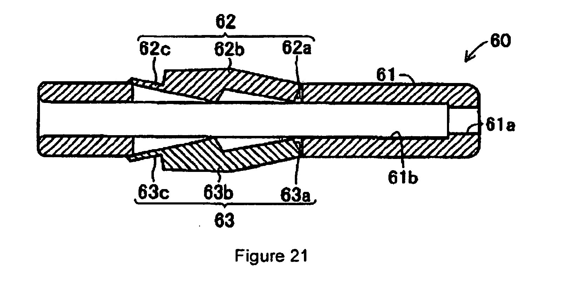

Then, base end portion (62a) of movable held part (62) is connected by a hinge to the rear end edge portion of window portion (64a), and base end portion (63a) of movable holding part (63) is connected by a hinge to the rear end edge portion of window portion (64b). In the state shown in Figures 20 and 21, outer side portion (62b) of movable held part (62) protrudes towards the outer side of main body (61). Also, flexible planar shaped gripping piece (62c) is formed on the front end (free end) of movable gripped part (62). In the state shown in Figures 20 and 21, outer side portion (63b) of movable holding part (63) protrudes towards the outer side of main body (61). On the front end (free end) of movable gripped part (63), flexible planar-shaped gripping piece (63c) is formed.

For said gripping pieces (62c), (63c), when the pair of movable gripped parts (62), (63) is energized towards the inner side of main body (61), the parts are bent while in contact with the front end edge portions of window portions (64a), (64b), and in this state, they enter main body (61). The structure is such that, after gripping pieces (62c), (63c) enter main body (61), by means of the front end edge portions of window portions (64a), (64b), gripping pieces (62c), (63c) can be prevented from exiting the main body (61). The constitution of the remaining portion of said indwelling needle C is the same as that in said indwelling needle A. Consequently, the same part numbers as those adopted for indwelling needle A are adopted, and they will not be explained again.

When said indwelling needle C is in use, the same operation as that for indwelling needle A is performed. In this case, too, just as in said Embodiments 1 and 2, tip portion (11 a) of external needle (10) indwells in the blood vessel of the patient, and from the state shown in Figure 17, internal needle (20) is pulled together with suction part (25) to the rear side of external needle (10), and the state becomes that shown in Figure 18. Then, after front end portion (21 a) of metal needle (21) is positioned in needle tip protective part (60) in housing (42), internal needle (20) is pulled towards the rear side, and the step (21 b) of metal needle (21) and the fine-diameter portion (61 a) of needle tip protective part (60) grip each other.

Then, internal needle (20) is pulled to the rear side and together with suction part (25), internal needle (20) is pulled back from external needle (10) while needle tip protective part (60) grips the tip side portion of metal needle (21). In this case, when metal needle (21) is positioned in the portion inside needle tip protective part (60) corresponding to movable gripped parts (62), (63), by means of metal needle (21), movable gripped parts (62), (63) are energized to the outer side of main body (61), and they are set along slope surface (16) within housing (12). Then, as needle tip protective part (60) grips metal needle (21), they start moving towards the outer side of housing (12). As a result, outer side portions (62b), (63b) of movable gripped parts (62), (63) are energized to the inner side of main body (61) by slope surface (16).

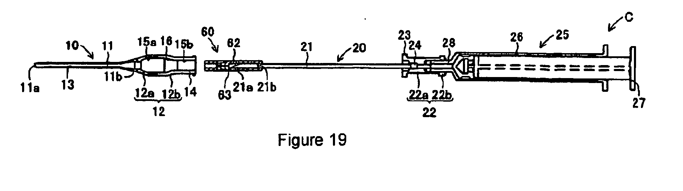

As a result, gripping pieces (62c), (63c) are in contact with the front end edge portions of window portions (64a), (64b) and are bent. In this state, they enter main body (61). Then, as internal needle (20) is pulled back together with suction part (25) to the rear side of external needle (10), while needle tip protective part (60) grips the tip of metal needle (21), internal needle (20) retreats together with suction part (25) from external needle (10), and the state becomes that shown in Figure 19. Then, the connecting portion of a tube member extending from the feeding device of the medicine solution or the like is connected to opening portion (14) of housing (12). In this way, the feeding device connected to the base end portion of the tuning means operates, and the medicine solution, etc., is fed to the blood vessel.

For indwelling needle C, when gripping pieces (62c), (63c) of said pair of movable gripped parts (62), (63) grip the edge portions of window portions (64a), (64b), the interior of main body (61) is obstructed while the gap formed between said pair of movable gripped parts (62), (63) becomes smaller than the diameter of metal needle (21). Consequently, there is no need to have gripping pieces (62c), (63c) grip each other, and the force for energizing said pair of movable gripped parts (62), (63) to the inner side of main body (61) can be small. The other functions and effects of indwelling needle C are the same as those of said indwelling needle A.

### Embodiment 4

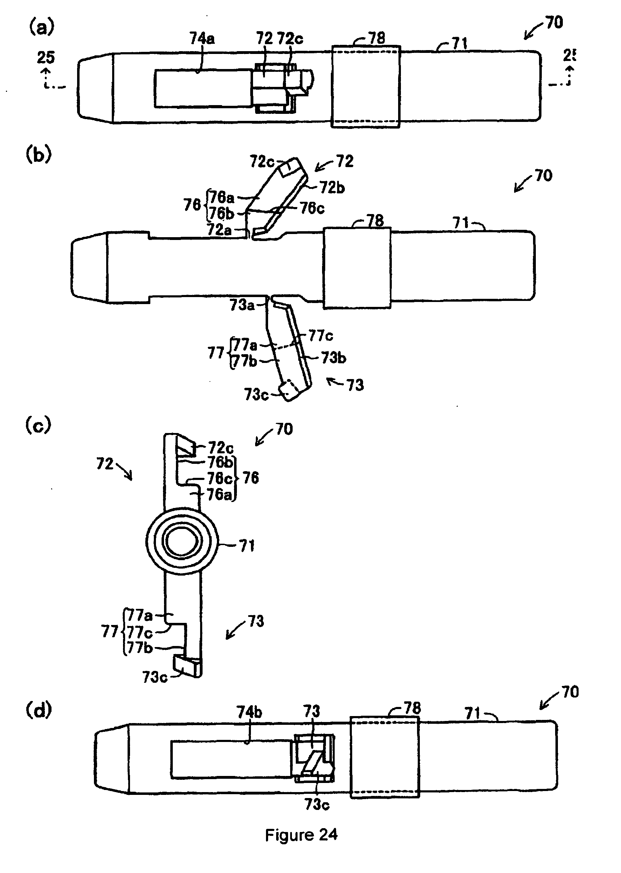

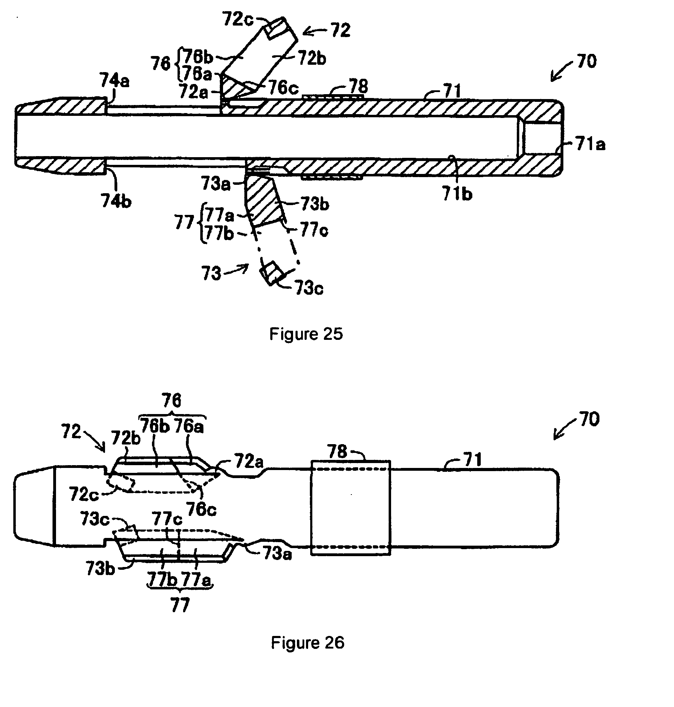

Figures 24 and 25 illustrate needle tip protective part (70) of the indwelling needle in Embodiment 4 of the present invention. Said needle tip protective part (70) is composed of main body (71) and a pair of movable gripped parts (72), (73). Main body (71) is formed in a cylindrical shape, and the inner peripheral surface of its rear end portion (the right hand side end portion shown in Figures 24 and 25) is formed with a diameter smaller than the remaining portion to form fine-diameter portion (71 a), and a step is formed between fine-diameter portion (71 a) and the remaining front-side peripheral surface (71b). Also, window portions (74a), (74b) extending in the axial direction are formed in the portions facing each other at the site a little ahead of the center in the axial direction of main body (71). Both said window portions (74a), (74b) are formed as rectangular holes with different lengths in the longitudinal direction. While these window portions have the front end portions positioned at the same site in the axial direction, their rear end portions are located at different positions, that is, the rear end portion of window portion (74b) is behind that of window portion (74a).

Then, base end portion (72a) of movable held part (72) is connected by a hinge to the rear end edge portion of window portion (74a), and base end portion (73a) of movable holding part (73) is connected by a hinge to the rear end edge portion of window portion (74b). Main body (76) of the gripping portion of movable gripped part (72) is composed of thicker portion (76a) on the side of base end portion (72a), and thinner portion (76b) on the tip side. The width of thicker portion (76a) is selected to be a little smaller than that of window portion (74a). The width of thinner portion (76b) is selected to be smaller such that one surface of thinner portion (76b) is positioned corresponding to nearly the center of window portion (74a) in the width direction.

Consequently, step (76c) is formed at the boundary between thicker portion (76a) and thinner portion (76b) on one surface of main body (76) of the gripping portion. Also, the thickness of thinner portion (76b) is selected to be a little smaller than half the thickness of thicker portion (76a). Also, in the state shown in Figure 26, outer side portion (72b) of movable gripped part (72) protrudes to the outer side of main body (71). Also, wedge-shaped gripping protrusion (72c) is formed on the surface on the side of step (76c) in thinner portion (76b) of the main body (76) of the gripping portion.

Also, main body (77) of the gripping portion of movable gripped part (73) is composed of thicker portion (77a) on the side of base end portion (73a) and thinner portion (77b) on the tip side. The width of thicker portion (77a) is selected to be a little smaller than that of window portion (74b). The width of thinner portion (77b) is selected to be smaller so that the other surface of thinner portion (77b) is located at nearly the center of window portion (74b) in the width direction. Consequently, step (77c) is formed at the boundary portion between thicker portion (77a) and thinner portion (77b) on the other surface of main body (77) of the gripping portion. The thickness of thinner portion (77b) is selected to be a little smaller than half the thickness of thicker portion (77a). Also, in the state shown in Figure 26, outer side portion (73b) of movable gripped part (73) protrudes towards the outer side of main body (71). On the surface on the side of step (77c) in thinner portion (77b) of main body (77) of the gripping portion, wedge-shaped gripping protrusion (73c) that can grip by gripping protrusion (72c) of movable gripped part (72) is formed.

On the outer peripheral surface of main body (71), ring-shaped member (78) is attached such that it can move in the axial direction of main body (71). As a result, as shown in Figures 26-31, as ring-shaped member (78) is moved from the rear end side to the front end side of main body (71), movable gripped parts (72), (73) can pass through the interior of window portions (74a), (74b) and can be pressed into main body (71). Also, on the rear end of the housing of the external needle having needle tip protective part (70) attached to it, a recess (not shown in the figure) for fixing ring-shaped member (78) is formed. The other features of the structure of the indwelling needle having said needle tip protective part (70) are the same as those of said indwelling needle A, and the same part numbers are adopted here.

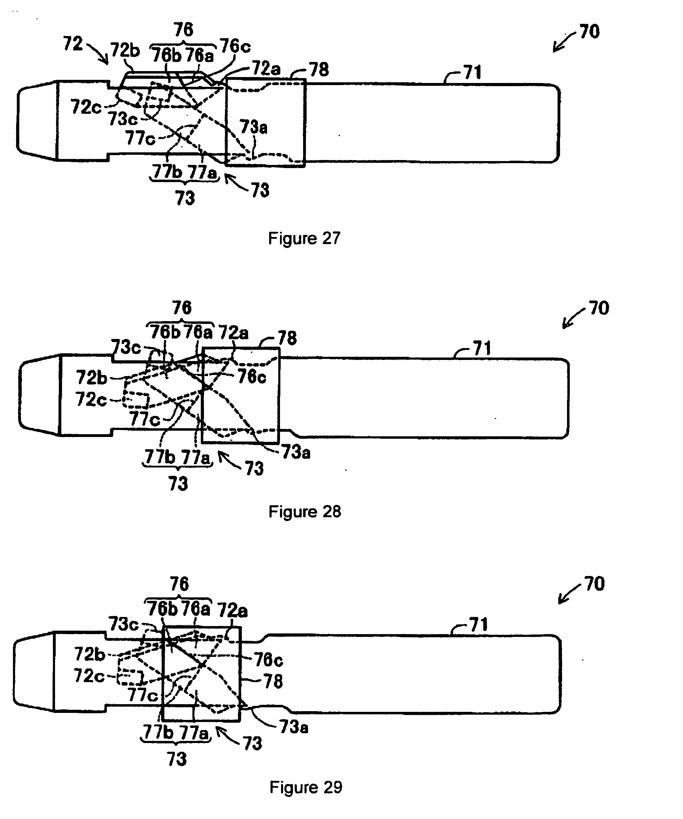

The state of gripping of movable gripped parts (72), (73) in the indwelling needle having said needle tip protective part (70) can be explained with reference to Figures 26-31. First of all, in the state shown in Figure 26 (when in use, it is accommodated in the housing of the external needle, with the metal needle inserted into the interior), ring-shaped member (78) is moved to the front side of main body (71), and the state becomes that shown in Figure 27. As a result, after the front end portion of ring-shaped member (78) comes into contact with base end portion (73a) of movable gripped part (73), movable gripped part (73) is pressed ahead. As a result, movable gripped part (73) rotates around base end portion (73a) at the center, and it enters main body (71). In this case, as shown in Figure 27, thinner portion (77b) and wedge-shaped gripping protrusion (73c) of movable gripped part (73) move to enter the recess side portion of thinner portion (76b) of movable gripped part (72).

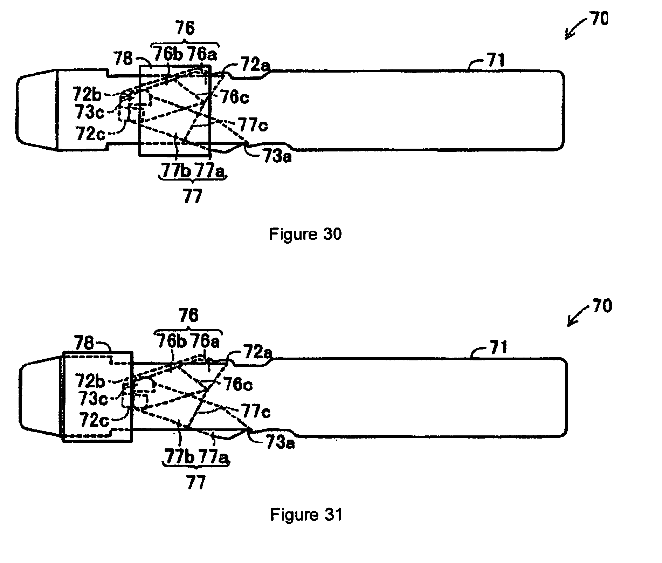

When ring-shaped member (78) is further moved towards the front side of main body (71), after the tip portion of ring-shaped member (78) comes into contact with base end portion (72a) of movable gripped part (72), it presses movable gripped part (72) forward. As a result, movable gripped part (72) rotates around base end portion (72a) at the center and, as shown in Figure 28, it enters main body (71). In this case, gripping protrusion (72c) of movable gripped part (72) moves so that it detours to the outer side of wedge-shaped gripping protrusion (73c) of movable gripped part (73). Then, as shown in Figure 29, the rear end portion of ring-shaped member (78) passes through the portion of main body (71) corresponding to base end portion (72a) of movable gripped part (72). Then, the rear end portion of ring-shaped member (78) is made to pass through the portion of main body (71) corresponding to outer side portion (72b) of movable gripped part (72), and the state becomes that shown in Figure 30.

As a result, gripping protrusion (72c) of movable gripped part (72) and gripping protrusion (73c) of movable gripped part (73) become located near the central axis of main body (71), and their positions in the axial direction are nearly the same. Consequently, gripping protrusion (72c) and gripping protrusion (73c) grip each other. Then, as shown in Figure 31, even when ring-shaped member (78) is moved to the front side of main body (71), the gripping state between gripping protrusion (72c) and gripping protrusion (73c) is maintained. That is, the gripping state of gripping protrusion (72c) and gripping protrusion (73c) is maintained as long as the gripping position is not away from the central axis of main body (71) over a prescribed distance.

In this case, the length of ring-shaped member (78) in the axial direction is increased, and in the state shown in Figure 31, the rear end portion of ring-shaped member (78) can also be located behind the gripping position between gripping protrusion (72c) and gripping protrusion (73c). As a result, even when gripping protrusion (72c) and gripping protrusion (73c) are disengaged from each other, because movable gripped parts (72), (73) are covered by ring-shaped member (78), they do not protrude outside main body (71). Consequently, the needle tip of the internal needle can be protected and the stability becomes even higher. The operation for using the indwelling needle having said needle tip protective part (70) is the same as that in said embodiments, so that it will not be explained in detail again. Also, the same functions and effects as those in said embodiments can be realized for the indwelling needle having needle tip protective part (70).

The indwelling needle of the present invention is not limited to the aforementioned embodiments. One can make appropriate modifications to the embodiments. For example, in said embodiments, external needle (10) of indwelling needle A or the like indwells in the blood vessel. However, the sites for indwelling for the indwelling needle of the present invention are not limited to the blood vessels. They also include other portions in the patient's body, such as the thoracic cavity, pleural cavity, gallbladder and liver, renal pelvis, bladder, etc. In this case, it can be used not only in feeding medicine solutions, etc., to the prescribed portion in the human body, but also in removing body fluids from the prescribed portion in the body. Also, in said Embodiment 4, ring-shaped member (78) is used and movable gripped parts (72), (73) are energized to the inner side of main body (71). However, one may also adopt a scheme in which, instead of ring-shaped member (78), the interior of the housing is used as the pressing means. Similarly, one may also make use of the ring-shaped member as the pressing means in said Embodiments 1-3.

Although the above embodiments have been described as incorporating two movable holding parts, it will be realized that further embodiments may have more than two such movable parts, for example three or even four. Additionally, one movable holding part may be sufficient to trap the inner needle tip portion sufficiently to protect the user of the equipment, particularly if the one holding part has an interior surface shaped to as to engage with the tip portion.

In addition, various materials can be used in manufacturing indwelling needle A. For example, external needle (10), the portion other than metal needle (21) of internal needle (20), needle tip protective part (30), etc., can be made of polypropylene, polycarbonate, polyurethane, nylon, silicone, polyether imides, polyether ether ketones, ABS resins, polyethylene, and other materials. Also, cannula (11) may be made of fluororesins, polyurethane, and other materials. In addition, for the shapes and materials for forming indwelling needle A, etc., appropriate modifications can be made within the technical range of the present invention.

In the appended claims, "proximal" means away from the point of patient insertion, i.e. the right hand side in the figures and "distal" means towards to the point of patient insertion, i.e. the left hand side of the figures.

## Claims

1. An indwelling needle equipment (A, B, C, D) comprising:
an outer cannula (11) for indwelling placement in a patient, said cannula having a housing (12) attached to a proximal end thereof;
an inner needle (20) having a connection part (22) at a proximal end thereof and
a needle tip protector (30) positioned within said housing and around said inner needle wherein said needle tip protector comprises a first portion having a sidewall defining a through-hole of a first diameter and a second portion having a sidewall defining a through-hole of a second diameter, smaller than said first diameter and wherein said sidewall of said first portion comprises at least one blocking member (32, 33) attached thereto and inwardly movable,
said inner needle having a tip portion (21 a) and a main portion (21), said tip portion having a larger diameter than said main portion and wherein said tip portion, said main portion and said second portion are dimensioned such that said main portion but not said tip portion is slidable through said through-hole of said second portion
wherein said indwelling equipment is configured such that withdrawing said inner needle from said cannula in a proximal direction causes said tip portion (21 a) to engage with said second portion, thereby withdrawing said needle tip protector from said housing and causing said at least one blocking member (32, 33) to move inwardly into said first portion through-hole so as to trap said tip portion (21a) therein; and
wherein said needle tip protector (30) comprises two blocking members (32, 33) diametrically opposed to one another,
**characterized in that** said housing (12) and said blocking members are shaped such that on withdrawing said inner needle, said housing is arranged to exert inward pressure on said blocking members (32, 33) causing inward movement thereof.

2. The indwelling needle equipment according to claim 1 wherein said blocking members (32, 33) are attached to said needle tip protector first portion sidewall at a proximal end (32a, 33a) of said blocking member.

3. The indwelling needle equipment according to claim 2 wherein said blocking members (32, 33) have distal ends (32c, 33c) formed to as to provide a locking action when mutually engaged.

4. The indwelling needle equipment according to claim 1 wherein said needle tip protector (70) further comprises a slip ring (78) arranged such that on withdrawal of said inner needle said slip ring is caused to slide over said blocking members (72, 73) moving them inwardly.

5. The indwelling needle equipment according to claim 4 wherein said blocking members (72, 73) are attached to said needle tip protector first portion sidewall (71) at a proximal end of said blocking member.

6. The indwelling needle equipment according to claim 5 wherein said blocking members (72, 73) have distal ends formed to as to provide a locking action when mutually engaged.

7. The indwelling needle equipment according to claim 1 wherein said blocking members (52, 53) are attached to said needle tip protector first portion sidewall (51) at a distal end of said blocking member.

8. The indwelling needle equipment according to claim 7 wherein said two blocking members (52, 53) have proximal ends (52c, 53c) forming a tongue and groove interrelation.

## Patentansprüche

1. Verweilnadelausrüstung (A, B, C, D) mit
einer äußeren Kanüle (11) zur Verweilanordnung in einem Patienten, wobei an einem proximalen Ende der Kanüle ein Gehäuse (12) angebracht ist,
einer inneren Nadel (20) mit einem Verbindungsteil (22) an einem proximalen Ende davon und
einem Nadelspitzenschutz (30), der in dem Gehäuse und um die innere Nadel herum positioniert ist und einen ersten Abschnitt mit einer Seitenwand, die ein Durchgangsloch mit einem ersten Durchmesser definiert, und einen zweiten Abschnitt mit einer Seitenwand, die ein Durchgangsloch mit einem zweiten Durchmesser, der kleiner als der erste ist, umfasst, wobei die Seitenwand des ersten Abschnitts mindestens ein Blockierelement (32, 33) umfasst, das daran angebracht ist und nach innen bewegt werden kann,
wobei die innere Nadel einen Spitzenabschnitt (21 a) und einen Hauptabschnitt (21) hat, wobei der Spitzenabschnitt einen größeren Durchmesser als der Hauptabschnitt hat und der Spitzenabschnitt, der Hauptabschnitt und der zweite Abschnitt so bemessen sind, dass zwar der Hauptabschnitt, aber nicht der Spitzenabschnitt durch das Durchgangsloch des zweiten Abschnitts gleiten kann,
wobei die Verweilausrüstung so konfiguriert ist, dass durch Herausziehen der inneren Nadel aus der Kanüle in einer proximalen Richtung veranlasst wird, dass der Spitzenabschnitt (21a) mit dem zweiten Abschnitt in Eingriff kommt, wodurch der Nadelspitzenschutz aus dem Gehäuse herausgezogen wird und veranlasst wird, dass sich das mindestens eine Blockierelement (32, 33) nach innen in das Durchgangsloch des ersten Abschnitts bewegt, um den Spitzenabschnitt (21 a) darin einzufangen, und
wobei der Nadelspitzenschutz (30) zwei sich diametral gegenüberliegende Blockierelemente (32, 33) umfasst,
**dadurch gekennzeichnet, dass** das Gehäuse (12) und die Blockierelemente so geformt sind, dass das Gehäuse beim Herausziehen der inneren Nadel so angeordnet ist, dass es nach innen Druck auf die Blockierelemente (32, 33) ausübt, so dass diese sich nach innen bewegen.

2. Verweilnadelausrüstung nach Anspruch 1, wobei die Blockierelemente (32, 33) an ihrem proximalen Ende (32a, 33a) an der Seitenwand des ersten Abschnitts des Nadelspitzenschutzes angebracht sind.

3. Verweilnadelausrüstung nach Anspruch 2, wobei die Blockierelemente (32, 33) distale Enden (32c, 33c) haben, die so ausgebildet sind, dass sie in gegenseitigem Eingriff für eine Verriegelungswirkung sorgen.

4. Verweilnadelausrüstung nach Anspruch 1, wobei der Nadelspitzenschutz (70) ferner einen Gleitring (78) umfasst, der so angeordnet ist, dass veranlasst wird, dass der Gleitring beim Herausziehen der inneren Nadel über die Blockierelemente (72, 73) gleitet und diese nach innen bewegt.

5. Verweilnadelausrüstung nach Anspruch 4, wobei die Blockierelemente (72, 73) an ihrem proximalen Ende an der Seitenwand (71) des ersten Abschnitts des Nadelspitzenschutzes angebracht sind.

6. Verweilnadelausrüstung nach Anspruch 5, wobei die Blockierelemente (72, 73) distale Enden haben, die so ausgebildet sind, dass sie in gegenseitigem Eingriff für eine Verriegelungswirkung sorgen.

7. Verweilnadelausrüstung nach Anspruch 1, wobei die Blockierelemente (52, 53) an ihrem distalen Ende an der Seitenwand (51) des ersten Abschnitts des Nadelspitzenschutzes angebracht sind.

8. Verweilnadelausrüstung nach Anspruch 7, wobei die beiden Blockierelemente (52, 53) proximale Enden (52c, 53c) haben, die nach Nut-und-Feder-Art zusammenwirken.

## Revendications

1. Equipement d'aiguille à demeure (A, B, C, D) comprenant :
une canule externe (11) à placer à demeure dans un patient, ladite canule ayant un logement (12) attaché à son extrémité proximale ;
une aiguille interne (20) ayant une partie de connexion (22) à son extrémité proximale et
un protège-pointe d'aiguille (30) positionné dans ledit logement et autour de ladite aiguille interne, ledit protège-pointe d'aiguille comprenant une première portion ayant une paroi latérale définissant un trou traversant ayant un premier diamètre et une deuxième portion ayant une paroi latérale définissant un trou traversant ayant un deuxième diamètre plus petit que ledit premier diamètre, ladite paroi latérale de ladite première portion comprenant au moins un organe de blocage (32, 33) attaché à celle-ci et déplaçable vers l'intérieur,
ladite aiguille interne ayant une portion de pointe (21a) et une portion principale (21), ladite portion de pointe ayant un plus grand diamètre que ladite portion principale et ladite portion de pointe, ladite portion principale et ladite deuxième portion étant dimensionnées de telle sorte que ladite portion principale mais pas ladite portion de pointe puisse coulisser à travers ledit trou traversant de ladite deuxième portion,
ledit équipement à demeure étant configuré de telle sorte que le retrait de ladite aiguille interne de ladite canule dans une direction proximale provoque l'engagement de ladite portion de pointe (21 a) avec ladite deuxième portion, retirant ainsi ledit protège-pointe d'aiguille dudit logement et provoquant le déplacement dudit au moins un organe de blocage (32, 33) vers l'intérieur dans le trou traversant de ladite première portion de manière à y piéger ladite portion de pointe (21 a) ; et
ledit protège-pointe d'aiguille (30) comprenant deux organes de blocage (32, 33) diamétralement opposés l'un à l'autre,
**caractérisé en ce que** ledit logement (12) et lesdits organes de blocage sont formés de telle sorte que lors du retrait de ladite aiguille interne, ledit logement soit conçu pour exercer une pression vers l'intérieur sur lesdits organes de blocage (32, 33) en provoquant leur déplacement vers l'intérieur.

2. Equipement d'aiguille à demeure selon la revendication 1, dans lequel lesdits organes de blocage (32, 33) sont attachés à la paroi latérale de la première portion dudit protège-pointe d'aiguille à une extrémité proximale (32a, 33a) dudit organe de blocage.

3. Equipement d'aiguille à demeure selon la revendication 2, dans lequel lesdits organes de blocage (32, 33) ont des extrémités distales (32c, 33c) formées de manière à fournir une action de verrouillage lorsqu'elles sont engagées les unes dans les autres.

4. Equipement d'aiguille à demeure selon la revendication 1, dans lequel ledit protège-pointe d'aiguille (70) comprend en outre une bague coulissante (78) agencée de telle sorte qu'au retrait de ladite aiguille interne, ladite bague coulissante soit amenée à coulisser par-dessus lesdits organes de blocage (72, 73) en les déplaçant vers l'intérieur.

5. Equipement d'aiguille à demeure selon la revendication 4, dans lequel lesdits organes de blocage (72, 73) sont attachés à la paroi latérale (71) de la première portion dudit protège-pointe d'aiguille à une extrémité proximale dudit organe de blocage.

6. Equipement d'aiguille à demeure selon la revendication 5, dans lequel lesdits organes de blocage (72, 73) ont des extrémités distales formées de manière à fournir une action de verrouillage lorsqu'elles sont engagées les unes dans les autres.

7. Equipement d'aiguille à demeure selon la revendication 1, dans lequel lesdits organes de blocage (52, 53) sont attachés à la paroi latérale (51) de la première portion dudit protège-pointe d'aiguille à une extrémité distale dudit organe de blocage.

8. Equipement d'aiguille à demeure selon la revendication 7, dans lequel lesdits deux organes de blocage (52, 53) ont des extrémités proximales (52c, 53c) formant une interrelation à languette et encoche.
